# EUROPEAN PATENT SPECIFICATION

(11) **EP 2 335 598 B1**
(45) Date of publication and mention of the grant of the patent: **06.07.2016**
(21) Application number: 11154288.2
(22) Date of filing: 14.12.2007
(51) Int. Cl.: A61B 10/00, G01N 33/68

(54) **Method of analyzing for at least one allergy**
Verfahren zur Analyse mindestens einer Allergie
Procédé pour l'analyse d'au moins une allergie

(30) Priority: 21.12.2006 US 876452 P
(43) Date of publication of application: 22.06.2011
(62) Divisional of application: 07024260.7
(73) Proprietor: Bayer HealthCare LLC, Whippany, NJ 07981-0915 (US)
(72) Inventor: Rebec, Mihailo V., Bristol, IN 46507 (US)
(74) Representative: Cohausz & Florack

(56) References cited:
- WO-A-99/25826
- WO-A-02/076477
- WO-A-2007/008800
- US-A- 4 819 657
- US-A- 5 335 670
- US-A- 5 874 226
- US-A- 5 897 506
- US-A- 5 944 662
- MCBRIDE P ET AL: "Use of plasma histamine levels to monitor cutaneous mast cell degranulation.", THE JOURNAL OF ALLERGY AND CLINICAL IMMUNOLOGY FEB 1989, vol. 83, no. 2 Pt 1, February 1989 (1989-02), pages 374-380, XP002475419, ISSN: 0091-6749

## Description

### FIELD OF THE INVENTION

The present invention relates generally to a system to analyze the effect of least one allergen on skin and, more specifically, to a diffusion-based, continuous-monitoring system.

### BACKGROUND OF THE INVENTION

Allergies or Type I hypersensitivity are inflammations caused by unusual sensitivity to foreign substances. Inflammation is a complex process in which the body's defense system combats foreign entities. While the battle against foreign entities may be necessary for the body's survival, some defense systems respond to foreign entities, even innocuous ones, as dangerous and thereby damage surrounding tissue in the ensuing conflict. When a person is hypersensitized, these substances are known as allergens.

Atopic allergy or atopy is an ecogenetic disorder, where genetic background dictates the response to environmental stimuli, such as pollen, food, dander and insect venoms. This disorder is generally characterized by an increased ability of lymphocytes to produce IgE antibodies in response to ubiquitous allergens. Activation of the immune system by these allergens leads to allergic inflammation and may occur after ingestion, penetration through the skin or after inhalation.

The effect of foreign substances on individuals varies. Because of this variation, individuals are often tested for selected allergies. One type of test that is used for identifying an allergen that triggers an allergic reaction is a skin test (also referred to as a scratch test). In one test, a small amount of the suspected allergen is placed on the skin. The skin is then gently scratched with a sterile needle. For example, if a specific food allergy is suspected, a skin test uses a dilute liquid extract of the suspect food. A small drop of this particular extract is placed on the skin of the forearm or back. This underlying skin is gently scratched through the small drop with a sterile needle.

If the skin reddens and, more importantly, if it swells, then the test is read as positive and an allergy to that substance is considered probable. If there is no reaction, it is read as negative. If the skin test is positive, it implies that the patient has a type of antibody (e.g., an IgE antibody) on specialized cells in the skin that release histamines that cause redness and itching. This type of test has disadvantages because the reaction is very qualitative in nature and, thus, requires a sufficiently large exposure site to be observed. Thus, a mild reaction can be difficult to judge. Additionally, these tests involve a qualitative determination at one point in time, while most reactions are kinetic in nature. If the observation is not made in the presence of a physician, any time relationship of the reaction may be lost and the extent of the reaction may be difficult to determine.

In addition to skin tests, other tests may be performed to determine allergies. For example, a special blood test, such as the RAST or the ELISA, may be used to determine selected allergies. These tests measure the presence of specific types of IgE in the blood. These tests tend to be more costly than skin tests and the results are not available immediately. Additionally, these tests involve a qualitative determination at one point in time, while most reactions are kinetic in nature. As with skin testing, positive RAST and ELISA tests do not by themselves necessarily result in a final diagnosis.

McBride, P. et al, Use of plasma histamine levels to monitor cutaneous mast cell degranulation, The Journal of Allergy and Clinical Immunology, Feb. 1989, vol. 83, pages 374-380, discloses a minimally invasive procedure for monitoring cutaneous mast cell degranulation in vivo in man. The body fluid in which the histamine level is detected is the plasma of venous blood. The blood is gathered by a butterfly catheter placed in a forearm vein during the testing. From the butterfly catheter blood samples are drawn for analysis. However, the concentrations of histamine in plasma of venous blood are very likely different from histamine concentrations in a layer of the skin

It would be desirable to have an improved method of analyzing for allergies.

### SUMMARY OF THE INVENTION

The subject-matter of the present invention is the diffusion-based, continuous-monitoring system of claim 1.

### BRIEF DESCRIPTION OF THE DRAWINGS

FIG. 1 is a diffusion-based, continuous-monitoring system shown in a transdermal application according to one embodiment.
FIG. 2 is the continuous-monitoring system of FIG. 1 being connected to a receiving module.

### DETAILED DESCRIPTION OF THE ILLUSTRATED EMBODIMENTS

The present invention is directed to a diffusion-based, continuous-monitoring system to analyze the reaction, if any, of an area of the skin exposed to an allergen. Thus, you are not analyzing for the allergen, but actually analyzing the reaction to the allergen. An allergen is defined herein as being a substance that causes a reaction.

By continuously monitoring information related to at least one antibody, histamines, leukotrienes and/or prostaglandins at an area of skin, the area of the skin can be analyzed to determine if an allergic reaction or inflammation is occurring and how severe the reaction or inflammation is. Antibodies are defined herein as proteins produced in the blood and tissue to assist in neutralizing and destroying foreign bodies (antigens). Examples of antibodies include, but are not limited to, Immunoglobulin E (IgE), Immunoglobulin G (IgG), Immunoglobulin A (IgA) and IgM antibody (Anti-HAV IgM). Antigens are defined herein as chemical substances (usually proteins) that the body perceives as foreign in which antigens causes the body to form antibodies against it when introduced into the body. Antigens may be other substances including, but not limited to, toxins, bacteria, foreign blood cells, and the cells of transplanted organs.

The phrase "information relating to antibodies, histamines, leukotrienes and/or prostaglandins" includes production, existence, localization, rate of release and/or release time of antibodies, histamines, leukotrienes and/or prostaglandins. This information may include amounts, relative concentrations, absolute concentrations and ratios to assist in determining the effect of an allergen on the skin. The term "information" as defined herein also includes changes in the amount, relative and absolute concentrations, and ratios whether in a percentage or absolute context. These "information" changes may be used over a selected duration of time such as, for example, a time change in amount, concentration or ratio. The "level" may refer to a time change in amount, concentration or ratio and compared to a later time change.

The allergens to be analyzed in, for example, body fluids like ISF (interstitial fluid), whole blood sample, intracellular and intercellular fluids. The allergens are often analyzed in ISF because the mode of transportation of most allergic reactions is initially through the ISF before proceeding to the blood. For example, if the skin comes into contact with poison ivy, the allergic reaction is initially through the ISF before it gets into the blood. Thus, it would be desirable to analyze the allergens in the ISF for poison ivy.

The information (e.g., production) related to the at least one antibody, histamine, leukotriene and/or prostaglandins at an area of skin is identified and then quantified. The quantity of antibodies, histamines, leukotrienes and/or prostaglandins produced may vary. In one example, an extract of a specific food may be applied to the skin (e.g., back) by creating small pin perforations such that the food extract is transported into the body. After the extract is applied, then the production of an antibody, histamine, leukotriene and/or prostaglandins is continuously monitored at the area of skin to determine if there is a reaction to the food extract. Many foods may be tested and some non-limiting common food allergies include eggs, peanuts, milk, nuts, soy, fish, wheat, peas and shellfish.

In another example, an extract of a leaf or the leaf itself may be applied to the skin by creating small pin perforations such that the leaf extract is transported into the body. After the extract is applied, then the production of an antibody, histamine, leukotriene and/or prostaglandins is continuously monitored at the area of skin to determine if there is a reaction to the leaf extract.

By identifying allergens to a particular individual, such allergens can be avoided or at least minimized. Additionally, if the allergens are severe, the particular individuals may have an appropriate remedy nearby to assist in reducing the effects of the allergic reaction. Additionally, an individual may assist medical personnel in identifying the cause of the reaction so as to assist in treating the allergic reaction. The individual may also be better able to quantify any such allergens and the timing of the allergic effect (how quickly the reaction occurs after exposure to the allergen).

In addition to food and leaves, it is contemplated that the skin may be tested for other allergens including, but not limited to, venom from insect stings (e.g., bees or wasps), pollens (e.g., grass, hay fever, trees and weeds), molds, animal dander and saliva, chemicals, dust mites, medicines, certain plants (e.g., poison ivy and poison oak) and other substances.

According to one embodiment, at least three criteria may be considered in selecting a suitable diffusion-based, continuous-monitoring system to analyze the production of at least one antibody, histamine, leukotriene and/or prostaglandins in a body fluid sample from an area of skin caused by a reaction to an allergen. First, a diffusion-enhancing process for the skin is selected. Second, a material is selected to assist in maintaining contact with the skin and further enhance diffusion of the at least one antibody, histamine, leukotriene and/or prostaglandins in the body fluid sample from an area of skin. Third, a diffusion-based, continuous-monitoring embodiment is selected to analyze the production of the at least one antibody, histamine, leukotriene and/or prostaglandins in the body fluid sample that diffuses from the skin.

According to one embodiment, the diffusion-enhancing process for the skin is selected based on factors such as the following: length of time of testing; the antibody, histamine, leukotriene and/or prostaglandins to be analyzed or monitored; and the area of the skin from where the antibody, histamine, leukotriene and/or prostaglandins is located. It is desirable for the diffusion-enhancing process to maintain the diffusion channel throughout the desired time period.

Skin abrasion is typically selected when the continuous-testing period is a relatively short period of time (e.g., less than about 8 hours). Skin abrasion is desirable for a shorter continuous-testing period because of the minimum impact on the skin. It is contemplated that a number of skin-abrasion techniques may be used. In one technique, skin abrasion occurs using a gel material including pumas or other skin-abrasion materials. In this technique, the gel material including pumas or other skin-abrasion materials is rubbed on the skin to increase the permeability of the skin. Skin abrasion may occur by other techniques such as using a generally coarse material (e.g., sandpaper), tape peeling or pumas paper.

To increase the porosity of skin (e.g., the stratum cornium, epidermis and/or dermis), chemical agents and physical agents may be used. The chemical and physical agents desirably assist in breaking down the lipids on the stratum cornium. The chemical and physical agents are typically used in short-term solutions and medium-term solutions. It is contemplated, however, that the chemical and physical agents may be used in long-term solutions.

The chemical agents may be skin hydration or skin exfoliates that increase the hydration and porosity of the skin. Skin hydration/exfoliates may include those commercially used in skin products. Some non-limiting examples of chemical agents that may be used include d-limonene, L-limonene, and alpha-terpinene. These chemical agents act by extracting lipids from, for example, the stratum cornium, which disrupts the stratum cornium and desquamates stratum cornium flake.

There are number of physical processes that can be used to enhance the permeability of the skin so as to increase the diffusion of the monitored antibody, histamine, leukotriene and/or prostaglandins of interest. In one process, needle-less jet injectors are used with very fine, particulates of inert material that are fired directly into the skin using high-pressure gas. In another process, pulsed magnetic fields may be used to create transient pores in the skin, resulting in increased permeation. It is contemplated that other physical processes may be used to enhance the permeability of the skin.

If the continuous-testing period is longer (e.g., from about 8 hours to 24 hours), then a different diffusion-enhancing approach may be selected. For such a period, various approaches may be selected such as microporation, microneedle-diffusion enhancement, pressure members, multiple lances, heavier abrasions and ultrasound energy.

In one embodiment, a microporation or a microneedle-diffusion enhancement approach may be used for longer continuous testing periods. A microporation approach creates sub-millimeter size apertures in the epidermis. In one microporation technique, a laser-poration technique may be used to deliver laser power directly to the skin to create apertures or pores. Laser-poration techniques are typically used to form shallow apertures or pores.

In a further embodiment, a series of absorbing dots is located in the stratum cornium and then followed by delivery of a laser that absorbs and softens at each point. The absorbent material converts the laser power to heat, which combined with pressure, create the apertures in the stratum cornium.

A microneedle-diffusion enhancement approach creates apertures in the epidermis and dermis. In another embodiment, a pressure member is adapted to apply pressure to and stretch the skin in preparation for forming a tear in the skin. In another approach, a more heavier abrasion of the skin could be performed such as using a more coarse material. An example of a more coarse material includes, but is not limited to, a coarser sandpaper.

In another embodiment, ultrasound energy is used to disrupt the lipid bilayer of the stratum cornium so as to increase the skin permeability. Ultrasound energy typically forms shallow apertures. By increasing the skin permeability, the amount of interstitial fluid (ISF) used in monitoring the production of antibodies, histamines and/or leukotrienes. One non-limiting source of an ultrasound energy system is Sontra SonoPrep® ultrasonic skin permeation system marketed by Sontra Medical Corporation. The SonoPrep® system applies relatively low frequency ultrasonic energy to the skin for a limited duration (from about 10 to 20 seconds). The ultrasonic horn contained in the device vibrates at about 55,000 times per second (55KHz) and applies energy to the skin through the liquid medium (e.g., hydrogel or liquid) to create cavitation bubbles that expand and contract in the liquid medium.

The chemical and physical agents discussed above in the generally short term can also be used in medium continuous-testing periods to increase and maintain the porosity of the skin. It is contemplated, however, that the chemical and physical agents may be used to obtain longer term action. For example, delipidating agents may be used in combination with physical agents such as ultrasonic preparation to create more long term diffusional channels.

If the continuous-testing period is even longer (e.g., at least 24 hours to about 48 hours), a deep, laser-ablation technique or lance may be selected. A deep, laser-ablation technique is desirable because the monitoring process can function longer due to the time needed to close the aperture created in the skin. The laser-ablation technique typically forms wide apertures. It is contemplated that a microneedle diffusion-enhancing approach, laser poration or lancets may also be used to provide a deeper aperture.

The size of the antibody, histamine, leukotriene and/or prostaglandins to be analyzed may also affect the diffusion-enhancing technique to be used. Typically, since many antibodies (e.g., IgG, IgM and IgE) are large molecules, the diffusion-enhancing process would desirably form a larger aperture in the skin. Typically, IgA and histamines are smaller than IgG, IgM and IgE molecules but are still relatively large and, thus, the diffusion-enhancing process may still desirably form a medium-sized aperture in the skin.

The area of the skin where the antibody, histamine, leukotriene and/or prostaglandins is located is also a consideration in selecting the diffusion-enhancing process. For example, if the epidermis or the upper part of the dermis is where the antibody, histamine, leukotriene and/or prostaglandins is to be monitored, the diffusion-enhancing process would be selected to disrupt the stratum cornium. Examples of such diffusion-enhancing processes include skin abrasion, skin hydrations (which increase the hydration of the skin) and skin exfoliates.

If monitoring of the antibodies, histamines, leukotrienes and/or prostaglandins in the ISF of the lower dermis is desired, the diffusion-enhancing process is selected to create diffusion channels deep into the dermis. If monitoring of the antibodies, histamines, leukotrienes and/or prostaglandins in the ISF in the subcutaneous region is desired, the diffusion-enhancing process is selected to create diffusion channels through the dermis into the subcutaneous region. Non-limiting examples of diffusion-enhancing processes that create deep diffusion channels into the dermis or subcutaneous region include, but are not limited to, laser poration, microneedles and lancets. It is also contemplated that an electric discharge with high energy and conductivity may also be used to create deep diffusion channels.

The chemical and physical agents discussed above in the generally short term may also be used in longer continuous-testing periods to increase and maintain the porosity of the skin.

In addition to selecting a continuous diffusion-enhancing method, a material is selected to assist in maintaining contact with the skin and to match the monitoring requirements in one embodiment. The diffusion-enhancing material maintains desirable skin contact at all times and assists in maintaining the diffusion channel. The material may be selected based on factors such as the following: length of monitoring time; the antibody, histamines, leukotrienes and/or prostaglandins to be monitored; and the area of the skin from where the antibody, histamines, leukotrienes and/or prostaglandins is located. For example, the viscosity of the material may be matched with the antibody, histamine, leukotriene and/or prostaglandins to be monitored.

Examples of diffusion-enhancing materials that may be used in the diffusion-based, continuous monitoring system include, but are not limited to, hydrogels, liquids and a liquid-stabilizing layer containing a liquid or hydrogel. The diffusion-enhancing material also desirably assists in hydrating the skin and maintaining an opening in the skin. By maintaining the opening, a liquid bridge is formed such that the antibody, histamines, leukotrienes and/or prostaglandins diffuses from a layer in the skin through the opening. The liquid bridge may be between a hydrogel/liquid and a body fluid such as ISF (interstitial fluid) or a whole blood sample.

The hydrogels typically have high water content and tacky characteristics. Hydrogels assist in (a) carrying the antibody, histamine, leukotriene and/or prostaglandins to the continuous-monitoring system; (b) carrying the allergen to the skin surface; and (c) hydrating the skin. Hydrogels are typically used with smaller sized antibody, histamines, leukotrienes and/or prostaglandins, shorter analysis times and an upper dermis analysis site.

A hydrogel composition is defined herein as including a cross-linked polymer gel. The hydrogel composition generally comprises at least one monomer and a solvent. The solvent is typically substantially biocompatible with the skin. Non-limiting examples of solvents that may be used in the hydrogel composition include water and a water mixture. The amount of solvent in the hydrogel is generally from about 10 to about 95 weight percent and may vary depending on the monomer amount, crosslinking, and/or the desired composition of the gel. One non-limiting example of a hydrogel/liquid is dimethylsulfoxide (DMSO). DMSO also assists in solubilizing lipids. An example of a liquid that may be used include an alcohol in combination with water. The chemical agents discussed above may be added to the hydrogel composition to maintain the porosity of the skin. It is contemplated that other hydrogels/liquids may be used.

The hydrogel/liquid may be located in a material (i.e., a liquid-stabilizing layer). This material may be selected to assist in maintaining contact with the skin as well as being able to retain the hydrogel/liquid. The liquid-stabilizing layer may include a chamber where the antibody/histamine/leukotriene/ prostaglandins of interest can diffuse. One non-limiting example of a material that can be used is a sponge or spongy material. The spongy material includes unbound liquid such as water and provides some structure to the unbound water. The spongy material is typically used with larger-sized antibodies, histamines, leukotrienes and/or prostaglandins, longer monitoring times, and deeper monitoring sites.

Other materials may be used to create content with skin and conduct further analysis. Materials include, but are not limited to, woven materials, non-woven materials, and polymeric films with apertures or porations formed therein. The polymeric films may be, for example, cast polymeric films. These materials may be used with liquids to facilitate diffusion of the material from the skin.

The amount of hydrogel that is selected is based on the need to provide a hydrated skin and having the hydrogel remain in intimate contact with the skin. One disadvantage of using a large amount of hydrogel is the potential impact on the lag time of the antibody/histamines/leukotrienes/ prostaglandins diffusing to the diffusion-based, continuous-monitoring system and/or the allergen reaching the skin. Such occurrences may potentially impact on the analysis time.

In addition to the allergens discussed above, additives may be added to the hydrogel or liquid. For example, to assist in dissolving lipids, the hydrogel or liquid may include SDS (sodium dodecyl (lauryl) sulfate) or SLS (sodium lauryl (laureth) sulfate). It is contemplated that other additives may be included in the hydrogel or liquid to assist in dissolving the lipids such as soaps. In another embodiment, DMSO may be used as an additive to another hydrogel/liquid to assist in solubilizing lipids.

Additional analysis components may also be added to the hydrogels/liquids. More specifically, additives may be added to the hydrogels/liquid to assist in monitoring the delivery of the allergen.

In another embodiment, an interference-filtering component may be added to the hydrogels/liquids. These interference-filtering components may include size exclusion, interference-binding molecules, and/or molecules that remove or convert interfering substances. One type of non-limiting interference-filtering components include membranes with molecular weight cutoffs that are intended to keep high molecular proteins from the analysis. For example, such interference-filtering components may be used to differentiate an IgM from a IgG response. Some non-limiting examples of interference-binding molecules are materials with appropriate charges. Another example is changing the ionic charge nature of the hydrogel or diffusion matrix such that charged interference molecules are inhibited from getting to the surface of the continuous-monitoring device.

Hypertonic solutions, hypotonic solutions and buffered solutions may be used as a diffusion-enhancing material. Hypertonic solutions are solutions having a high solute concentration, while hypotonic solutions are solutions having a low solute concentration. Hypertonic solutions assist in driving up the body fluid (e.g., ISF) closer to the skin surface. Hypotonic solutions, on the other hand, assist in driving up the antibodies/histamines/leukotrienes/prostaglandins closer to the skin surface. The hypertonic or hypotonic solutions in one embodiment may be included with the hydrogel or liquid.

It is contemplated that other additives such an anticoagulants and/or buffers may be used to assist in maintaining the localized pH near the optimal level. It is contemplated that other additives may be added to the hydrogel or liquid to assist in monitoring the production of a specific antibody/histamine/leukotriene/prostaglandins.

A diffusion-based, continuous-monitoring device is selected that analyzes the production of antibodies/histamines/leukotrienes/prostaglandins of the body fluid sample that is diffused from the skin. The diffusion-based, continuous-monitoring device may be selected from an electrochemical-monitoring system, an optical-monitoring system, an osmotic-monitoring system and a pressure-based monitoring system. A pressure-based monitoring system includes systems associated with the binding of an antibody/histamine/leukotriene/prostaglandins by components of the hydrogel, which results in a volume change in the gel. The monitoring may be performed in a vertical or horizontal direction with respect to the diffusion channel(s) formed in the skin. It is contemplated that the antibodies/histamines/leukotrienes/prostaglandins may be carried out in the material that is selected to assist in maintaining contact with the skin (e.g., the hydrogel or liquid).

The diffusion-based, continuous-monitoring device is typically located near or at the skin. The diffusion-based, continuous-monitoring device may be coupled with the skin and is typically in intimate contact with the skin. For example, the diffusion-based, continuous-monitoring device may be adhered to the skin with an adhesive. The adhesive may be the hydrogel itself. In another embodiment, the adhesive is a separate component whose sole function is to adhere the continuous-monitoring device to the skin. In a further embodiment, the diffusion-based, continuous-monitoring device may be coupled to the skin by a mechanical attachment. For example, the mechanical attachment may be a wrist band (e.g., an elastic band, a watch band, a band with an attachment mechanism such as a hook and loop mechanism). One example of a hook and loop mechanism is a Velcro® strap marketed by 3M Corporation of St. Paul, Minnesota. It is contemplated that other mechanical attachments may be used to couple or attach the continuous-monitoring device with skin.

The diffusion-based, continuous-monitoring device may have a variety of forms. For example, the continuous-monitoring device may be a pad, circular disk, polygonal shaped or non-polygonal shaped. The continuous-monitoring system may include an analysis element. For example, a pad with an analysis element may be used instead of, or in addition to, the analysis element being initially located in the hydrogel or liquid. In one embodiment, an enzyme may be initially located in the continuous-monitoring device.

In one embodiment, the diffusion-based, continuous-monitoring device includes a processor to process the data, a memory that stores data, and a communications interface. The data may be stored at regular intervals such as, for example, every minute, every 5 minutes or every 30 minutes. The intervals may be shorter such as every second or longer such as being several hours apart. It is contemplated that other regular or non-regular intervals may be used to store the data.

The data may be any information that assists in monitoring the production of antibodies/histamines/leukotrienes/prostaglandins. This may include the level of antibody/histamine/leukotriene/prostaglandins, the amount of antibody/histamine/leukotriene/prostaglandins released, the rate of antibody/histamine/leukotriene/prostaglandins released, the duration of the release of antibody/histamine/leukotriene/prostaglandins, the ratios of selected antibody/histamine/leukotriene/prostaglandins to other substances. By storing the data in the continuous-monitoring device, this data can be accessed and used to assist in monitoring information related to the antibodies/histamines/leukotrienes/prostaglandins. It is desirable for the continuous-monitoring device to tabulate, transmit and store information that assists in analyzing the effect of an allergen on the skin.

In one embodiment, the continuous-monitoring device is connected to a remote-monitoring system over a communications link. The communications link between the continuous-monitoring device and the remote-monitoring system may be wireless, hard wired or a combination thereof. The wireless communications link may include an RF link, an infrared link or an inductive magnetic link. The wireless implementation may include an internet connection. The continuous-monitoring device may communicate via its communication interface with devices such as a computer, e-mail server, cell phone or telephone. It is contemplated that the continuous-monitoring device may include other devices that are capable of storing, sending and/or receiving information.

A remote-monitoring system enables an individual such as a physician to monitor the production of antibodies, histamines, leukotrienes and/or prostaglandins from a remote location. The remote-monitoring system may be located in, for example, a hospital. The physician may be able to access information from the continuous-monitoring device via its communications interface using, for example, a computer or telephone. The remote-monitoring system is especially desirable for patients who are less lucid and need assistance with monitoring production of antibodies/histamines/leukotrienes/prostaglandins. It is desirable for the remote-monitoring system to be able to display, calibrate and store information received from the communications interface.

The remote-monitoring system may be used to send back instructional information to the patients. In such an embodiment, diffusion-based continuous-monitoring device includes a communications link that has a receiver component to receive instructions from the remote-monitoring system in addition to a transmitter component to transmit information to the remote-monitoring system.

In one embodiment, the continuous-monitoring device may forward information over a communications link in real-time. In another embodiment, the continuous-monitoring device may store and process the data before forwarding the information over a communications link in another embodiment.

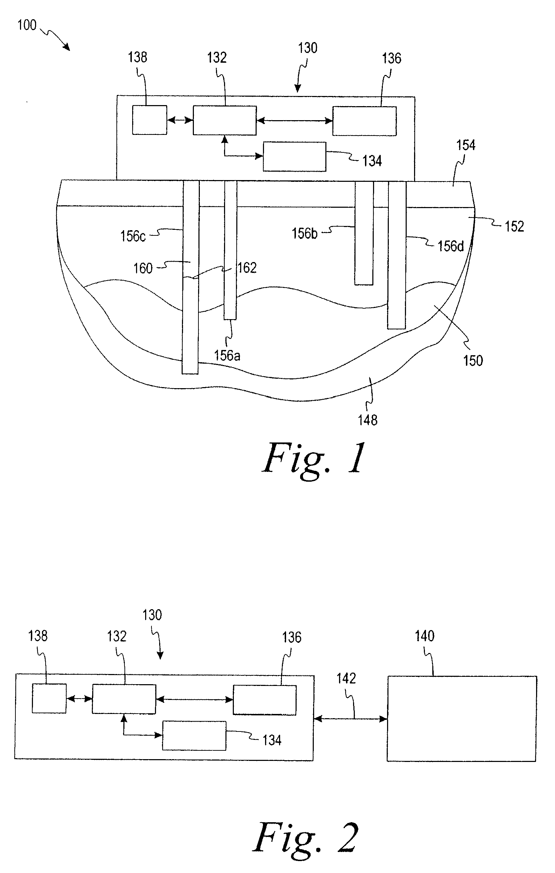

Referring to FIG. 1, a diffusion-based, continuous-monitoring system 100 is shown in a transdermal application. The continuous monitoring system 100 includes a continuous-monitoring device 130 being placed above skin. The continuous-monitoring device 130 of FIG. 1 includes a processor 132, memory 134, a communication interface 136 and an analysis component 138. Referring to FIG. 2, the continuous-monitoring device 130 is shown in communication with a receiving module 140 (e.g., a remote-monitoring station) over a communications link 142.

The skin as shown in FIG. 1 includes a subcutaneous layer 148, a dermis layer 150, an epidermis layer 152 and a stratum cornium layer 154. The stratum cornium layer 154 has a plurality of channels 156a-d formed therein. The plurality of channels 156a-d may be formed by different methods such as discussed above. The channels may be of different sizes and depths depending on the antibodies/histamines/leukotrienes/prostaglandins being analyzed and the location of the antibodies/histamines/leukotrienes/prostaglandins in the skin. The antibody/histamine/leukotriene/prostaglandins of interest may be located in the different layers of the skin. The antibody/histamine/leukotriene/prostaglandins of interest are primarily located in the dermis layer 150 or the subcutaneous layer 148. The hydrogel/liquid assists in diffusing the antibody/histamines/leukotrienes/prostaglandins to the surface of the skin.

In one embodiment, a hydrogel/liquid is used to assist in diffusing the antibodies/histamines/leukotrienes/prostaglandins to the surface of the skin. The channel 156c is shown with hydrogel/liquid 160. An interface 162 is formed between the hydrogel/liquid and the body fluid. The analysis may be performed in several locations in the continuous-monitoring system 100. For example, the analysis may be performed using the analysis components 138 in the continuous-monitoring device 130. The analysis components may include components such as a sensor, an enzyme or reagent, potentiostat, electrochemical analysis components (e.g., plurality of electrodes, etc.) and/or optical analysis components (e.g., light source, detector, etc.). In another example, the analysis may be performed on the skin and/or in the channels. It is contemplated that the analysis may take place in more than one location. For example, the hydrogel/liquid may include an analysis portion (e.g., a reagent or enzyme) that reacts with antibodies/histamines/leukotrienes/prostaglandins in the channel, while the remainder of the analysis takes place on the skin or in the continuous-monitoring device 130.

According to one process, a technician programs the diffusion-based, continuous-monitoring device for operation. The technician may program, for example, the antibody/histamine/leukotriene/prostaglandins to be monitored, the length of time of the monitoring, rate of when the data is to be collected, when the response is completed, and when the device can be removed. The technician may then proceed to form apertures in the skin that function as diffusion channels as discussed above for the desired time period. The skin is contacted with at least one selected allergen. It is desirable to contact the skin with the at least one selected allergen at or near the diffusion channel. It is contemplated, however, that the skin may be contacted with the at least one selected allergen in a location away from the diffusion channels. The skin may be contacted with the at least one selected allergen using different methods. One non-limiting example is to place a sensor pad with at least one selected allergen on the skin. The technician locates the continuous-monitoring device on the individual. In one method, the technician locates the continuous-monitoring device on the arm. It is contemplated that the technician may locate the continuous-monitoring device on other locations. The continuous-monitoring device is adapted to process, calibrate, display, store and/or transmit information related to the antibody/histamine/leukotriene/prostaglandins.

## Claims

1. A diffusion-based, continuous-monitoring system for analyzing the effect of at least one allergen on a patient, the diffusion-based, continuous-monitoring system comprising:
means for creating at least one diffusion channel in an area of skin of the patient for diffusing at least one of an antibody, a histamine, a leukotriene, and prostaglandins from the skin, wherein the means for creating at least one diffusion channel include at least one of the following: means for performing skin abrasion, means for performing microporation, means for performing microneedle-diffusion enhancement, pressure members, lancets, means for performing ultrasound, and means for performing laser ablation;
means for maintaining the at least one diffusion channel for a desired continuous duration of time, wherein the means for maintaining the at least one diffusion channel includes chemical agents or physical agents, or both, that increase and maintain porosity of the area of skin;
means for applying at least one selected allergen to the area of skin, wherein the means for applying at least one selected allergen to the area of skin are means for contacting the skin with at least one allergen at or near the at least one diffusion channel;
means for continuously monitoring information relating to at least one of an antibody, a histamine, a leukotriene, and prostaglandins at the area of skin for the desired continuous duration of time wherein the means for continuously monitoring information is a diffusion-based, continuous-monitoring device, comprising
an analysis component for analyzing at least one of an antibody, a histamine, a leukotriene, and prostaglandins that is diffused from the skin through the at least one diffusion channel, the analysis component being adapted to continuously monitor at least one of amounts, relative concentrations, absolute concentrations, and ratios for at least one of an antibody, a histamine, a leukotriene, and prostaglandins for the desired continuous duration of time to identify a quantity of the at least one allergen at which the at least one allergen causes an allergic effect, the analysis component including at least one of the following: an electrochemical-monitoring system, an optical-monitoring system, an osmotic-monitoring system, and a pressure-monitoring system; and
means for analyzing the information relating to the at least one of an antibody, a histamine, a leukotriene, and prostaglandins at the area of skin to identify a quantify of the at least one selected allergen at which the at least one selected allergen causes an allergic effect, the means for analyzing the information comprising a processor for processing the at least one of amounts, relative concentrations, absolute concentrations, and ratios for the at least one of an antibody, a histamine, a leukotriene, and prostaglandins.

2. The system of claim 1, wherein the means for creating the at least one diffusion channel is operable for creating a plurality of diffusion channels.

3. The system of claim 1, wherein the desired duration is a continuous time period of at least approximately 8 hours.

4. The system of claim 1, wherein the means for continuously monitoring information is an electrochemical diffusion-based, continuous-monitoring device.

5. The system of claim 1, wherein the means for continuously monitoring information is an optical diffusion-based, continuous-monitoring device.

6. The system of claim 1, wherein the means for analyzing the information is adapted to analyze information related to the antibody at the area of skin.

7. The system of claim 1, wherein the means for analyzing the information is adapted to analyze information related to the histamine at the area of skin.

8. A diffusion-based, continuous-monitoring system of claim 1, further comprising a memory for storing the at least one of amounts, relative concentrations, absolute concentrations, and ratios for the at least one of an antibody, a histamine, a leukotriene, and prostaglandins.

9. The system of claim 8, wherein the at least one diffusion channel is a plurality of diffusion channels.

10. The system of one of claim 8 or 9, wherein the diffusion-based, continuous-monitoring device is a pad.

11. The system of one of the claims 8 to 10, wherein the diffusion-based, continuous-monitoring device is adapted to be coupled to the skin by means of an adhesive or by mechanical attachment.

12. The system of claims 11, wherein the adhesive includes a hydrogel.

13. The system of claims 11, wherein the mechanical attachment includes a band.

14. The system of claim 8, wherein the desired duration is at least 8 hours.

15. The system of one of the claims 8 or 14, wherein the desired duration is at least 24 hours.

16. The system of one of the claims 8 to 15, wherein the antibody is at least one of Immunoglobulin E (IgE), Immunoglobulin G (IgG), Immunoglobulin A (IgA) or IgM antibody (Anti-HAV IgM).

17. The system of one of the claims 8 to 16, wherein the diffusion-based, continuous-monitoring device further comprising means for displaying the information related to the at least one of an antibody, a histamine, a leukotriene, and prostaglandins.

18. The system of one of the claims 8 to 17, wherein the information includes at least one of production, rate of release, and release time of the at least one of an antibody, a histamine, a leukotriene, and prostaglandins.

19. The system of one of the claims 8 to 18, further comprising a hydrogel or liquid topographically applicable on the skin to assist in enhancing the diffusion of the at least one of an antibody, a histamine, a leukotriene, and prostaglandins, wherein the diffusion-based, continuous monitoring device is adapted to be positioned in communication with the hydrogel or liquid.

20. The system of claim 19, wherein the hydrogel or liquid includes a diagnostic element to assist in analyzing production, release rate, or both, of the at least one of an antibody, a histamine, a leukotriene, and prostaglandins.

21. The system of one of the claims 8 to 20, further comprising a receiving module and a communications link, wherein the diffusion-based, continuous monitoring device is adapted to connect with the receiving module via the communications link.

22. The system of claim 21, further being adapted to transmit information related to the at least one of an antibody, a histamine, a leukotriene, and prostaglandins via the communications link to the receiving module.

23. The system of claim 21, further being adapted to receive instructions from the receiving module via the communications link directed to the information related to the at least one of an antibody, a histamine, a leukotriene, and prostaglandins.

24. The system of one of the claims 21 to 23, wherein the system is adapted to transmit information between the diffusion-based, continuous monitoring device and the receiving module at intervals between approximately 5 minutes and approximately 2 hours.

## Patentansprüche

1. Diffusionsbasiertes, kontinuierliches Überwachungssystem zum Analysieren der Wirkung mindestens eines Allergens auf einen Patienten, wobei das diffusionsbasierte, kontinuierliche Überwachungssystem umfasst:
Mittel zum Erzeugen mindestens eines Diffusionskanals in einem Hautareal des Patienten zum Diffundieren von mindestens einem von einem Antikörper, einem Histamin, einem Leukotrien und Prostaglandinen von der Haut, wobei die Mittel zum Erzeugen mindestens eines Diffusionskanals mindestens eines der folgenden umfasst: Mittel zum Durchführen von Hautabschürfung, Mittel zum Durchführen von Mikroporation, Mittel zum Durchführen von Mikronadeldiffusionsverstärkung, Druckelemente, Lanzetten, Mittel zum Durchführen von Ultraschall und Mittel zum Durchführen von Laserablation;
Mittel zum Aufrechterhalten des mindestens einen Diffusionskanals für eine gewünschte kontinuierliche Zeitdauer, wobei die Mittel zum Aufrechterhalten des mindestens einen Diffusionskanals chemische Mittel oder physikalische Mittel oder beide umfassen, welche die Porosität des Hautareals erhöhen und aufrechterhalten;
Mittel zum Anwenden mindestens eines ausgewählten Allergens auf das Hautareal, wobei die Mittel zum Anwenden mindestens eines ausgewählten Allergens auf das Hautareal Mittel zum Inberührungbringen der Haut mit mindestens einem Allergen an dem einen Diffusionskanal oder in dessen Nähe sind;
Mittel zum kontinuierlichen Überwachen von Information bezüglich mindestens eines von einem Antikörper, einem Histamin, einem Leukotrien und Prostaglandinen an dem Hautareal für die gewünschte kontinuierliche Zeitdauer, wobei das Mittel zum kontinuierlichen Überwachen von Information eine diffusionsbasierte, kontinuierliche Überwachungsvorrichtung ist, umfassend
eine Analysekomponente zum Analysieren von mindestens einem von einem Antikörper, einem Histamin, einem Leukotrien und Prostaglandinen, der/das von der Haut durch den mindestens einen Diffusionskanal diffundiert, wobei die Analysekomponente eingerichtet ist, um mindestens eines von Mengen, relativen Konzentrationen, absoluten Konzentrationen und Verhältnissen für mindestens eines von einem Antikörper, einem Histamin, einem Leukotrien und Prostaglandinen für die gewünschte kontinuierliche Zeitdauer kontinuierlich zu überwachen, um eine Quantität des mindestens einen Allergens zu identifizieren, bei der das mindestens eine Allergen eine allergische Wirkung verursacht, wobei die Analysekomponente mindestens eines der folgenden umfasst: ein elektrochemisches Überwachungssystem, ein optisches Überwachungssystem, ein osmotisches Überwachungssystem und ein Drucküberwachungssystem; und
Mittel zum Analysieren der Informationen bezüglich des mindestens einen von einem Antikörper, einem Histamin, einem Leukotrien und Prostaglandinen an dem Hautareal, um eine Quantität des mindestens einen ausgewählten Allergens zu identifizieren, bei der das mindestens eine ausgewählte Allergen eine allergische Wirkung verursacht, wobei das Mittel zum Analysieren der Informationen einen Prozessor zum Verarbeiten der mindestens einen von Mengen, relativen Konzentrationen, absoluten Konzentrationen und Verhältnissen für das mindestens eine von einem Antikörper, einem Histamin, einem Leukotrien und Prostaglandinen aufweist.

2. System nach Anspruch 1, wobei das Mittel zum Erzeugen des mindestens einen Diffusionskanals zum Erzeugen mehrerer Diffusionskanäle eingerichtet ist.

3. System nach Anspruch 1, wobei die gewünschte Dauer ein kontinuierlicher Zeitraum von mindestens etwa 8 Stunden ist.

4. System nach Anspruch 1, wobei das Mittel zum kontinuierlichen Überwachen von Information eine elektrochemische, diffusionsbasierte, kontinuierliche Überwachungsvorrichtung ist.

5. System nach Anspruch 1, wobei das Mittel zum kontinuierlichen Überwachen von Information eine optische, diffusionsbasierte, kontinuierliche Überwachungsvorrichtung ist.

6. System nach Anspruch 1, wobei das Mittel zum Analysieren der Informationen eingerichtet ist, um Informationen bezüglich des Antikörpers an dem Hautareal zu analysieren.

7. System nach Anspruch 1, wobei das Mittel zum Analysieren der Informationen eingerichtet ist, um Informationen bezüglich des Histamins an dem Hautareal zu analysieren.

8. Diffusionsbasiertes, kontinuierliches Überwachungssystem nach Anspruch 1, ferner umfassend einen Speicher zum Speichern des mindestens einen von Mengen, relativen Konzentrationen, absoluten Konzentrationen und Verhältnissen für das mindestens eine von einem Antikörper, einem Histamin, einem Leukotrien und Prostaglandinen.

9. System nach Anspruch 8, wobei der mindestens eine Diffusionskanal mehrere Diffusionskanäle sind.

10. System nach einem von Anspruch 8 oder 9, wobei die diffusionsbasierte, kontinuierliche Überwachungsvorrichtung ein Pad ist.

11. System nach einem von Anspruch 8 bis 10, wobei die diffusionsbasierte, kontinuierliche Überwachungsvorrichtung eingerichtet ist, um mittels eines Klebstoffs oder mechanischer Befestigung mit der Haut gekoppelt zu werden.

12. System nach Anspruch 11, wobei der Klebstoff ein Hydrogel umfasst.

13. System nach Anspruch 11, wobei die mechanische Befestigung ein Band umfasst.

14. System nach Anspruch 8, wobei die gewünschte Dauer mindestens 8 Stunden beträgt.

15. System nach einem von Anspruch 8 oder 14, wobei die gewünschte Dauer mindestens 24 Stunden beträgt.

16. System nach einem von Anspruch 8 bis 15, wobei der Antikörper mindestens eines von Immunglobulin E (IgE), Immunglobulin G (IgG), Immunglobulin A (IgA) oder IgM-Antikörper (Anti-HAV-IgM) ist.

17. System nach einem von Anspruch 8 bis 16, wobei die diffusionsbasierte, kontinuierliche Überwachungsvorrichtung ferner Mittel zum Anzeigen der Informationen bezüglich des mindestens einen von einem Antikörper, einem Histamin, einem Leukotrien und Prostaglandinen aufweist.

18. System nach einem von Anspruch 8 bis 17, wobei die Information mindestens eines von Produktion, Freisetzungsgeschwindigkeit und Freisetzungszeit des mindestens einen von einem Antikörper, einem Histamin, einem Leukotrien und Prostaglandinen beinhalten.

19. System nach einem von Anspruch 8 bis 18, ferner umfassend ein Hydrogel oder eine Flüssigkeit, die topographisch auf die Haut anwendbar ist, um bei der Verstärkung der Diffusion des mindestens einen von einem Antikörper, einem Histamin, einem Leukotrien und Prostaglandinen zu unterstützen, wobei die diffusionsbasierte, kontinuierliche Überwachungsvorrichtung eingerichtet ist, um in Kommunikation mit dem Hydrogel oder der Flüssigkeit platziert zu werden.

20. System nach Anspruch 19, wobei das Hydrogel oder die Flüssigkeit ein diagnostisches Element umfasst, um beim Analysieren von Produktion, Freisetzungsgeschwindigkeit oder beidem des mindestens einen von einem Antikörper, einem Histamin, einem Leukotrien und Prostaglandinen zu unterstützen.

21. System nach einem von Anspruch 8 bis 20, ferner umfassend ein lämpfangsmodul und eine Kommunikationsverbindung, wobei die diffusionsbasierte, kontinuierliche Überwachungsvorrichtung eingerichtet ist, um sich über die Kommunikationsverbindung mit dem Empfangsmodul zu verbinden.

22. System nach Anspruch 21, ferner eingerichtet, um Informationen bezüglich des mindestens einen von einem Antikörper, einem Histamin, einem Leukotrien und Prostaglandinen über die Kommunikationsverbindung an das Empfangsmodul zu übermitteln.

23. System nach Anspruch 21, ferner eingerichtet, um über die Kommunikationsverbindung von dem Empfangsmodul Anweisungen zu empfangen, die auf die Informationen bezüglich des mindestens einen von einem Antikörper, einem Histamin, einem Leukotrien und Prostaglandinen gerichtet sind.

24. System nach einem von Anspruch 21 bis 23, wobei das System eingerichtet ist, zwischen der diffusionsbasierten, kontinuierlichen Überwachungsvorrichtung und dem Empfangsmodul in Zeitabständen zwischen etwa 5 Minuten und etwa 2 Stunden Informationen zu übermitteln.

## Revendications

1. Système de contrôle continu, basé sur la diffusion, analysant l'effet d'au moins un allergène sur un patient, le système de contrôle continu, basé sur la diffusion comprenant :
des moyens pour créer au moins un canal de diffusion sur une zone de peau du patient, pour faire diffuser au moins un parmi un anticorps, une histamine, un leucotriène, et des prostaglandines dans la peau, où les moyens pour créer au moins un canal de diffusion comprennent au moins l'un parmi : des moyens pour effectuer une abrasion de la peau, des moyens pour effectuer une microporation, des moyens pour effectuer une amplification de diffusion par micro-aiguille, des éléments de pression, des lancettes, des moyens pour effectuer des ultra-sons, et des moyens pour effectuer une ablation laser ;
des moyens pour maintenir le au moins un canal de diffusion pendant une période continue souhaitée, où les moyens pour maintenir le au moins un canal de diffusion comprennent des agents chimiques ou des agents physiques, ou les deux, qui augmentent et maintiennent la porosité de la surface de peau ;
des moyens pour appliquer au moins un allergène choisi sur la surface de peau, où les moyens pour appliquer au moins un allergène choisi sur la surface de peau sont des moyens de mise en contact de la peau avec au moins un allergène au niveau du ou près du au moins un canal de diffusion ;
des moyens pour contrôler en continu l'information concernant au moins un parmi un anticorps, une histamine, un leucotriène, et des prostaglandines sur la surface de peau pendant la période continue souhaitée, où les moyens pour le contrôle continu de l'information est un dispositif de contrôle continu, basé sur la diffusion, comprenant :
un composant d'analyse pour analyser au moins un parmi un anticorps, une histamine, un leucotriène, et des prostaglandine, qui sont diffusés dans la peau par au moins un canal de diffusion, le composant d'analyse étant destiné à contrôler en continu au moins l'un parmi les quantités, les concentrations relatives, les concentrations absolues et les rapports d'au moins un parmi un anticorps, une histamine, un leucotriène, et des prostaglandines pendant la période continue souhaitée, afin d'identifier la quantité du au moins un allergène, qui provoque l'effet allergique, le composant d'analyse comprenant au moins l'un des éléments suivants : un système de contrôle électrochimique, un système de contrôle optique, un système de contrôle osmotique et un système de contrôle de la pression, et
des moyens pour analyser l'information concernant le au moins un parmi un anticorps, une histamine, un leucotriène, et des prostaglandines sur la surface de peau, afin d'identifier la quantité du au moins un allergène, qui provoque un effet allergique, les moyens pour analyser l'information comprenant un processeur pour traiter au moins un parmi les quantités, les concentrations relatives, les concentrations absolues et les rapports d'au moins un parmi un anticorps, une histamine, un leucotriène, et des prostaglandines.

2. Système selon la revendication 1, où les moyens pour créer le au moins un canal de diffusion peut créer une série de canaux de diffusion.

3. Système selon la revendication 1, où la période souhaitée est une période continue d'au moins environ 8 heures.

4. Système selon la revendication 1, où les moyens pour le contrôle continu de l'information est un dispositif électrochimique de contrôle continu, basé sur la diffusion.

5. Système selon la revendication 1, où les moyens pour le contrôle continu de l'information est un dispositif optique de contrôle continu, basé sur la diffusion.

6. Système selon la revendication 1, où les moyens pour analyser l'information sont destinés à analyser l'information liée à l'anticorps sur la surface de la peau.

7. Système selon la revendication 1, où les moyens pour analyser l'information sont destinés à analyser l'information liée à l'histamine sur la surface de la peau.

8. Système de contrôle continu, basé sur la diffusion, selon la revendication 1, comprenant en outre, une mémoire pour stocker au moins un parmi les quantités, les concentrations relatives, les concentrations absolues et les rapports d'au moins un parmi un anticorps, une histamine, un leucotriène, et des prostaglandines.

9. Système selon la revendication 8, où le au moins un canal de diffusion est une série de canaux de diffusion.

10. Système selon la revendication 8 ou 9, où le dispositif de contrôle continu, basé sur la diffusion, est une compresse.

11. Système selon l'une des revendications 8 à 10, où le dispositif de contrôle continu, basé sur la diffusion est destiné à être couplé à la peau à l'aide d'un adhésif ou par fixation mécanique.

12. Système selon la revendication 11, où l'adhésif comprend un hydrogel.

13. Système selon la revendication 11, où la fixation mécanique comprend une bande.

14. Système selon la revendication 8, où la période souhaitée s'élève à au moins 8 heures.

15. Système selon l'une des revendications 8 à 14, où la période souhaitée s'élève à au moins 24 heures.

16. Système selon l'une des revendications 8 à 15, où l'anticorps est une immunoglobuline E (IgE), une immunoglobuline G (IgG), une immunoglobuline A (IgA) et/ou un anticorps IgM (IgM anti-VHA).

17. Système selon l'une des revendications 8 à 16, où le dispositif de contrôle continu, basé sur la diffusion comprend en outre, des moyens d'affichage de l'information liée à au moins un parmi un anticorps, une histamine, un leucotriène, et des prostaglandines.

18. Système selon l'une des revendications 8 à 17, où l'information comprend au moins l'un parmi la production, la vitesse de libération et le temps de libération du au moins un parmi un anticorps, une histamine, un leucotriène, et des prostaglandines.

19. Système selon l'une des revendications 8 à 18, comprenant en outre, un hydrogel ou un liquide applicable sur la peau pour aider à amplifier la diffusion du au moins un parmi un anticorps, une histamine, un leucotriène, et des prostaglandines, où le dispositif de contrôle continu, basé sur la diffusion est destiné à être positionné en communisation avec l'hydrogel ou le liquide.

20. Système selon la revendication 19, où l'hydrogel ou le liquide comprend un élément diagnostique pour aider à analyser la production, la vitesse de libération ou les deux, du au moins un parmi un anticorps, une histamine, un leucotriène, et des prostaglandines.

21. Système selon l'une des revendications 8 à 20, comprenant en outre, un module de réception et des liens de communication, où le dispositif de contrôle continu, basé sur la diffusion peut être connecté au module de réception via le lien de communication.

22. Système selon la revendication 21, destiné en outre, à transmettre l'information liée à au moins un parmi un anticorps, une histamine, un leucotriène, et des prostaglandines via le lien de communication au module de réception.

23. Système selon la revendication 21, destiné en outre, à recevoir des instructions du module de réception via le lien de communication, concernant l'information liée à au moins un parmi un anticorps, une histamine, un leucotriène, et des prostaglandines.

24. Système selon l'une des revendications 21 à 23, où le système est destiné à transmettre l'information entre le dispositif de contrôle continu, basé sur la diffusion et le module de réception à intervalles allant d'environ 5 minutes à environ 2 heures.
